# EUROPEAN PATENT SPECIFICATION

(11) **EP 4 504 305 B1**
(45) Date of publication and mention of the grant of the patent: **18.02.2026**
(21) Application number: 23711753.6
(22) Date of filing: 24.03.2023
(51) Int. Cl.: A61M 5/32, A61M 5/34

(54) **A SUB-ASSEMBLY OF A MEDICAMENT DELIVERY DEVICE**
UNTERANORDNUNG FÜR EINE MEDIKAMENTENABGABEVORRICHTUNG
SOUS-ENSEMBLE D'UN DISPOSITIF D'ADMINISTRATION DE MÉDICAMENTS

(30) Priority: 08.04.2022 EP 22167383
(43) Date of publication of application: 12.02.2025
(73) Proprietor: SHL Medical AG, 6302 Zug (CH)
(72) Inventor: CARLSSON, Daniel, 131 28 Nacka Strand (SE); ALEXANDERSSON, Oscar, 131 28 Nacka Strand (SE); ZANDER, Johan, 131 28 Nacka Strand (SE); YIN, Ming-Ting, Taoyuan City, 338 (TW)
(86) International application number: PCT/EP2023/057738
(87) International publication number: WO 2023/194132

(56) References cited:
- EP-A1- 2 544 741
- EP-B1- 2 544 741
- WO-A1-2020/015986
- WO-A1-2020/120087
- CN-B- 103 228 306
- US-A- 4 613 329
- US-A1- 2015 343 155

## Description

### TECHNICAL FIELD

The present disclosure generally relates to a sub-assembly of a medicament delivery device, and particularly to a sub-assembly with a rotation mechanism.

### BACKGROUND

Medicament delivery devices such as pen type manual injectors or auto-injectors are generally known for the self-administration of a medicament by patients without formal medical training. For example, patients suffering from diabetes may require repeated injections of insulin, or patients may require regular injections of other types of medicaments such as a growth hormone.

A medicament container, such as a cartridge or a syringe, is often inserted or pre-installed in a part of the medicament delivery device. Cartridges usually are more suitable for storing medicament for long period than syringes, as cartridges can keep the contained medicament being fully sealed until a user plans to carry out a medicament delivery operation. However, cartridges require the user to attach medicament delivery members, e.g., needles or nozzles, before use, which might be difficult for the user without formal medical training.

Therefore, a medicament delivery device that can attach the medicament delivery member automatically is desirable. EP 2 544 741 A1 discloses a medicament delivery device comprising a delivery member hub received in a tubular cap, wherein the hub is attached to a housing by relative rotation and a guard sleeve is axially movable. In this arrangement, the functions of hub and cap are integrated, without a separate adapter as an intermediate member.

### SUMMARY

The invention is defined by the appended claims, to which reference should now be made.

In the present disclosure, when the term "distal direction" is used, this refers to the direction pointing away from the dose delivery site during use of the injection device. When the term "distal part/end" is used, this refers to the part/end of the delivery device, or the parts/ends of the members thereof, which during use of the injection device is/are located furthest away from the dose delivery site. Correspondingly, when the term "proximal direction" is used, this refers to the direction pointing towards the dose delivery site during use of the injection device. When the term "proximal part/end" is used, this refers to the part/end of the delivery device, or the parts/ends of the members thereof, which during use of the injection device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", or "circumferentially" refer to a circumference or a circumferential direction relative to an axis, typically a central axis extending in the direction of the longest extension of the device and/or component. Similarly, "radial" or "radially" refer to a direction extending radially relative to the axis, and "rotation", "rotational" and "rotationally" refer to rotation relative to the axis.

There is hence provided a sub-assembly of a medicament delivery device, the sub-assembly comprising: a tubular cap, an adapter, a delivery member assembly, and a delivery member guard. The tubular cap extends along a longitudinal axis between a proximal end and a distal end. The delivery member assembly comprises a delivery member hub, and a delivery member attached to the delivery member hub. The delivery member hub comprises a fastener configured to be attached to a counter fastener of a housing of the medicament delivery device via a relative rotation between the fastener and the counter fastener. The delivery member hub is rotatable relative to the housing of the medicament delivery device about the longitudinal axis between an unattached position where the fastener is not attached to the counter fastener and an attached position where the fastener is attached to the counter fastener. The adapter comprises a body operably connected to the delivery member hub, so that the adapter is rotationally immovable and axially movable relative to the delivery member hub. The adapter is arranged within the tubular cap. The adapter comprises a distally directed surface. The delivery member guard comprises a proximally directed surface adjacent to the distally directed surface of the adapter. The delivery member guard is axially movable relative to the tubular cap from a distal position to a proximal position. A rotation mechanism is arranged between the tubular cap and the adapter. The rotation mechanism is configured so that a rotation of the adapter about the longitudinal axis relative to the tubular cap is triggered when the delivery member guard moves from the distal position to the proximal position, so that the delivery member hub is moved from the unattached position to the attached position by the rotation of the adapter.

Preferably, according to another embodiment, the rotation mechanism comprises a screw thread and a protrusion positioned within the screw thread. One of the screw thread and the protrusion extends from the body of the adapter. The other one of the screw thread and the protrusion extends from a body of the tubular cap towards the body of the adapter.

Preferably, according to another embodiment, the screw thread is arranged on an outer surface of the body of the adapter. The tubular cap comprises a distally extending arm extending from the body of the tubular cap in the direction of the longitudinal axis. The protrusion extends in a direction transverse to the longitudinal axis from the distally extending arm.

Alternatively, according to another embodiment, the screw thread is arranged on an inner surface of the body of the tubular cap. The protrusion extends in a direction transverse to the longitudinal axis from an outer surface of the body of the adapter.

Alternatively, according to another embodiment, the rotation mechanism comprises a torsion spring winding around the longitudinal axis. The torsion spring extends between a first end and a second end. The first end is fixed to the tubular cap. The second end is fixed to the body of the adapter. The adapter comprises an engaging member extending from the body of the adapter. The tubular cap comprises a counter engaging member aligned with the engaging member of the adapter in a circumferential direction when the delivery member guard is in the distal position and offset relative to the engaging member of the adapter in the circumferential direction when the delivery member guard is in the proximal position.

Preferably, according to another embodiment, the engaging member is a rib extending in the direction transverse to the longitudinal axis from the outer surface of the body of the adapter. The counter engaging member is a cut-out in a wall of the body of the tubular cap.

Preferably, according to another embodiment, the adapter comprises a flange extending from the body of the adapter. The flange defines the distally directed surface. A cut-out is arranged in the flange and wherein the second end of the torsion spring is attached within the cut-out of the flange.

Preferably, according to another embodiment, the delivery member assembly comprises a tubular inner cap. The delivery member hub is arranged within the tubular inner cap. The delivery member hub is rotationally immovable and axially movable relative to the inner cap via a first engagement between the delivery member hub and the inner cap. The inner cap is arranged within the body. The inner cap is rotationally immovable relative to the body of the adapter via a second engagement between the inner cap and the body of the adapter Preferably, according to another embodiment, the first engagement is formed by a longitudinally extending rib on the inner cap and a longitudinally extending rib on the delivery member hub.

Alternatively, according to another embodiment, the first engagement is formed by a longitudinally extending rib on the inner cap and a cut-out/recess on the delivery member hub, the cut-out/recess is open to the direction transverse to the longitudinal axis.

Alternatively, according to another embodiment, the first engagement is formed by a longitudinally extending rib on the delivery member hub and a cut-out/recess on the inner cap, the cut-out/recess is open to the direction transverse to the longitudinal axis.

Alternatively, according to another embodiment, the first engagement is formed by a non-circular cross-section body section of the delivery member hub matching with a non-circular cross-section body section of the inner cap.

Preferably, according to another embodiment, the second engagement is formed by a longitudinally extending rib on the inner cap and a longitudinally extending rib on the body of the adapter.

Alternatively, according to another embodiment, the second engagement is formed by a longitudinally extending rib on the inner cap and a cut-out/recess on the body of the adapter, the cut-out/recess is open to the direction transverse to the longitudinal axis.

Alternatively, according to another embodiment, the second engagement is formed by a longitudinally extending rib on the body of the adapter and a cut-out/recess on the inner cap, the cut-out/recess is open to the direction transverse to the longitudinal axis.

Alternatively, according to another embodiment, the second engagement is formed by a non-circular cross-section body section of the body of the adapter matching with a non-circular cross-section body section of the inner cap.

Preferably, according to another embodiment, the adapter is directly engaged with the delivery member hub via an engagement formed by a longitudinally extending rib on the delivery member hub and a longitudinally extending rib on the body of the adapter.

Alternatively, according to another embodiment, the adapter is directly engaged with the delivery member hub via an engagement formed by a longitudinally extending rib on the delivery member hub and a cut-out/recess on the body of the adapter, the cut-out/recess is open to the direction transverse to the longitudinal axis.

Alternatively, according to another embodiment, the adapter is directly engaged with the delivery member hub via an engagement formed by a longitudinally extending rib on the body of the adapter and a cut-out/recess on the delivery member hub, the cut-out/recess is open to the direction transverse to the longitudinal axis.

Alternatively, according to another embodiment, the adapter is directly engaged with the delivery member hub via an engagement formed by a non-circular cross-section body section of the body of the adapter matching with a non-circular cross-section body section of the delivery member hub.

Preferably, according to another embodiment, one of the fastener and the counter fastener is a screw thread, and the other one of the fastener and the counter fastener is a screw thread follower. The delivery member hub is axially movable relative to the housing of the medicament delivery device in the distal direction of the tubular cap.

Alternatively, according to another embodiment, one of the fastener and the counter fastener is a bayonet track and the other one of the fastener and the counter fastener is a bayonet track follower.

Preferably, according to another embodiment, the delivery member guard comprises a tubular body configured to surround the delivery member. The proximal end of the tubular body defines the proximally directed surface.

Another aspect of the invention provides a front sub-assembly for a medicament delivery device, the front sub-assembly comprising the sub-assembly. The front sub-assembly comprises a body extending along the longitudinal axis from a proximal end to a distal end. The delivery member guard is coaxially attached to the body and is axially movable relative to the body along the longitudinal axis, and a lock member is attached to the body. The lock member is movable relative to the delivery member guard between a locked position and an unlocked position. The lock member comprises a distally directed surface adjacent to a second proximally directed surface of the delivery member guard in the locked position. The distally directed surface of the lock member is spaced apart in a circumferential direction relative to the longitudinal axis from the proximally directed surface of the delivery member guard in the unlocked position.

Preferably, according to another embodiment, the lock member is rotatable relative to the delivery member guard around the longitudinal axis between the locked position and the unlocked position.

Preferably, according to another embodiment, the front sub-assembly, comprising a biasing member arranged between a distally directed surface of the delivery member guard and a proximally directed surface of the body.

Another aspect of the invention provides a medicament delivery device comprising the sub-assembly.

Another aspect of the invention provides a medicament delivery device comprising the front sub-assembly.

Preferably, according to another embodiment, the medicament delivery device comprising a multiple-chamber medicament container arranged within the carrier.

Preferably, according to another embodiment, the medicament delivery device is an auto-injector or a manual insulin pen.

Preferably, according to another embodiment, the medicament container is a cartridge.

Another aspect of the invention provides a method of attaching a delivery member to a medicament delivery device, the method comprising the following steps in the following order: providing: a tubular cap extending along a longitudinal axis; wherein the tubular cap houses an adapter; a delivery member hub; a delivery member fixed to the delivery member hub; a delivery member guard in contact with the adapter in the direction of the longitudinal axis; triggering a rotation of the adapter relative to the tubular cap about the longitudinal axis by using the delivery member guard to axially move the adapter in the direction of the longitudinal axis towards the tubular cap, thereby rotating the delivery member hub with the adapter relative to the tubular cap, and thereby attaching the delivery member to the medicament delivery device by the rotation of the delivery member hub.

Preferably, according to another embodiment, the step of triggering a rotation of the adapter relative to the tubular cap about the longitudinal axis by using the delivery member guard to axially move the adapter in the direction of the longitudinal axis comprising the steps of: using the delivery member guard to axially move the adapter out from a rotational block structure of the tubular cap; and rotating the adapter using a torsion spring that is fixed between the tubular cap and the adapter.

Preferably, according to another embodiment, the step of triggering a rotation of the adapter relative to the tubular cap about the longitudinal axis by using the delivery member guard to axially move the adapter in the direction of the longitudinal axis comprising the steps of: rotating the adapter using a converter assembly that is configured to convert the axial movement of the adapter into a rotational movement of the adapter.

Preferably, according to another embodiment, the converter assembly is formed by a thread and a protrusion positioned in the thread. One of the thread and the protrusion is positioned on an outer surface of the adapter and the other one of the thread and the protrusion is positioned on an inner surface of the tubular cap.

Preferably, according to another embodiment, the delivery member is attached to the medicament delivery device via a thread or bayonet connection between the delivery member hub and the medicament delivery device.

The injection devices described herein can be used for the treatment and/or prophylaxis of one or more of many different types of disorders. Exemplary disorders include, but are not limited to: rheumatoid arthritis, inflammatory bowel diseases (e.g. Crohn's disease and ulcerative colitis), hypercholesterolaemia, diabetes (e.g. type 2 diabetes), psoriasis, migraines, multiple sclerosis, anaemia, lupus, atopic dermatitis, asthma, nasal polyps, acute hypoglycaemia, obesity, anaphylaxis and allergies. Exemplary types of drugs that could be included in the injection devices described herein include, but are not limited to, antibodies, proteins, fusion proteins, peptibodies, polypeptides, pegylated proteins, protein fragments, protein analogues, protein variants, protein precursors, and/or protein derivatives. Exemplary drugs that could be included in the injection devices described herein include, but are not limited to (with non-limiting examples of relevant disorders in brackets): etanercept (rheumatoid arthritis, inflammatory bowel diseases (e.g. Crohn's disease and ulcerative colitis)), evolocumab (hypercholesterolaemia), exenatide (type 2 diabetes), secukinumab (psoriasis), erenumab (migraines), alirocumab (rheumatoid arthritis), methotrexate (amethopterin) (rheumatoid arthritis), tocilizumab (rheumatoid arthritis), interferon beta-1a (multiple sclerosis), sumatriptan (migraines), adalimumab (rheumatoid arthritis), darbepoetin alfa (anaemia), belimumab (lupus), peginterferon beta-1a' (multiple sclerosis), sarilumab (rheumatoid arthritis), semaglutide (type 2 diabetes, obesity), dupilumab (atopic dermatitis, asthma, nasal polyps, allergies), glucagon (acute hypoglycaemia), epinephrine (anaphylaxis), insulin (diabetes), atropine and vedolizumab (inflammatory bowel diseases (e.g. Crohn's disease and ulcerative colitis)). Pharmaceutical formulations including, but not limited to, any drug described herein are also contemplated for use in the injection devices described herein, for example pharmaceutical formulations comprising a drug as listed herein (or a pharmaceutically acceptable salt of the drug) and a pharmaceutically acceptable carrier. Pharmaceutical formulations comprising a drug as listed herein (or a pharmaceutically acceptable salt of the drug) may include one or more other active ingredients, or may be the only active ingredient present.

Furthermore, all terms used in the claims are to be interpreted according to their ordinary meaning in the technical field, unless explicitly defined otherwise herein. All references to "a/an/the element, apparatus, component, means, etc." are to be interpreted openly as referring to at least one instance of the element, apparatus, component, means, etc., unless explicitly stated otherwise.

### BRIEF DESCRIPTION OF THE DRAWINGS

Embodiments of the inventive concept will now be described, by way of example only, with reference to the accompanying drawings, in which:
Figs 1-3 schematically show perspective views of a delivery member assembly of the sub-assembly of the invention.
Figs 4A-4B schematically show an adapter of the invention.
Fig. 5 schematically shows a perspective view of the delivery member assembly of Figs 1-3 with the adapter of Figs 4A-4B.
Fig. 6 schematically shows a perspective view of a part of a tubular cap of the invention.
Fig. 7 schematically shows a cross-section view of the delivery member assembly of Figs 1-3 with the adapter of Figs 4A-4B and the tubular cap of the invention.
Fig. 8 schematically shows a perspective view of the delivery member assembly of Figs 1-3 with the adapter of the invention in another example and the tubular cap of the invention.
Fig. 9 schematically shows a perspective view of a delivery member guard of the invention.
Fig. 10 schematically shows a perspective view of the sub-assembly of the invention.
Fig. 11 schematically shows a perspective view of the sub-assembly of the invention in another example.
Fig. 12 schematically shows a perspective view of the adapter of the invention in another example and a torsion spring.
Figs 13-14 schematically show perspective views of the delivery member assembly of the tubular cap of the invention in the example of Fig. 12.
Fig. 15 schematically shows a perspective view of a medicament delivery device that can be used with the sub-assembly of the invention.
Figs 16A-16B schematically show perspective views of a lock member of the medicament delivery of Fig. 15.

### DETAILED DESCRIPTION

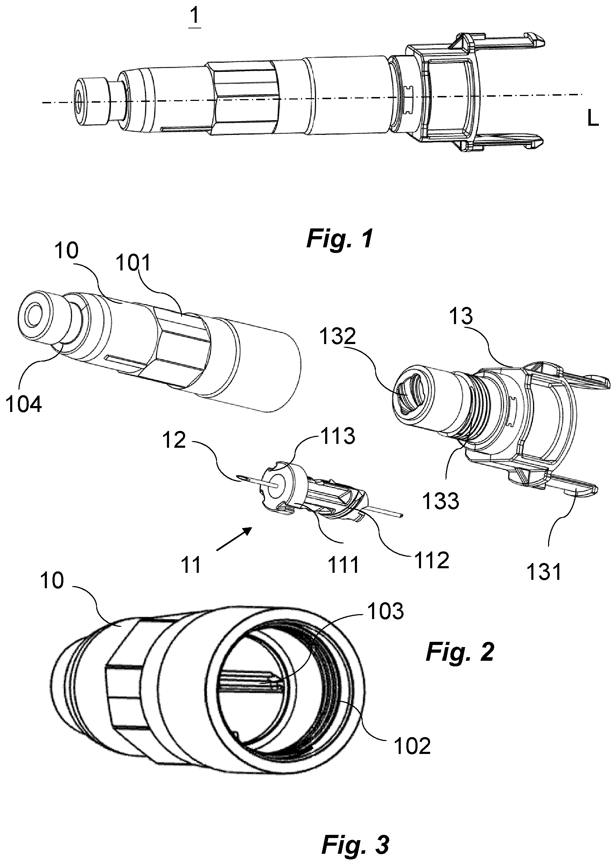

Figs 1-14 illustrates a sub-assembly of a medicament delivery device. The sub-assembly comprises a tubular cap 3; 3'; 3", an adapter 2; 2'; 2", a delivery member assembly 1, and a delivery member guard 4.

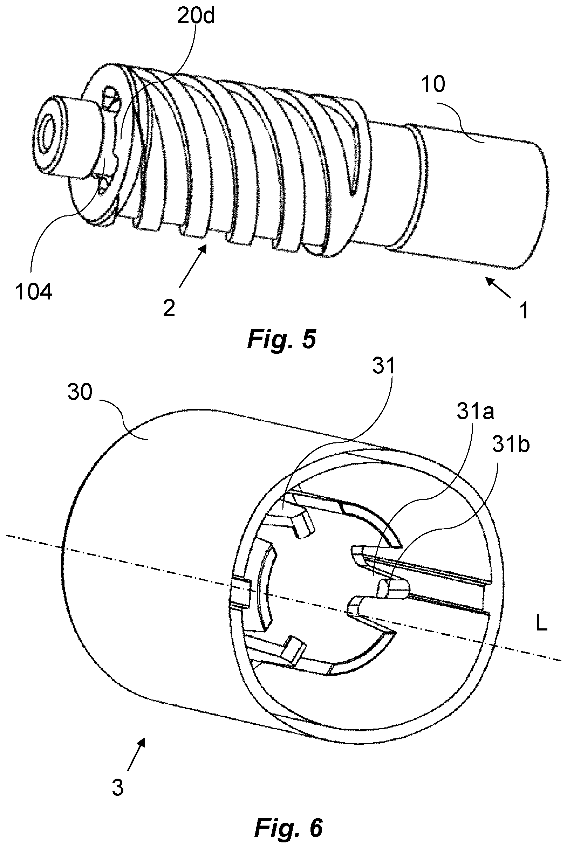

As shown in Fig. 6, the tubular cap 3; 3'; 3" extends along a longitudinal axis L between a proximal end and a distal end. The tubular cap 3; 3'; 3" comprises a body 30, 30', 30". The tubular cap 3; 3'; 3" is configured to be gripped by a user and is configured to be moved away from the medicament delivery device before a medicament delivery operation. As shown in Fig. 6, tubular cap 3; 3'; 3" is generally cylindrical. Alternatively, the body of the tubular cap may comprise a cross-section (viewing in the direction of the longitudinal axis) that is oval or polygonal, dependent on the design needs of the medicament delivery device.

As shown in Figs 1-3, the delivery member assembly 1 comprises a delivery member hub 11, and a delivery member 12 attached to the delivery member hub 11, e.g., via glue or a friction fit. The delivery member hub 11 comprises a fastener 112 configured to be attached to a counter fastener 132 of a housing of the medicament delivery device via a relative rotation between the fastener 112 and the counter fastener 132. The housing of the medicament delivery device is configured to accommodate a medicament of the medicament delivery device.

Therefore, when the delivery member hub 11 rotates relative to the housing of the medicament delivery device, the delivery member hub 11 is attached to the housing of the medicament delivery device via the fastener 112 and the counter fastener 132. Thus, a fluid communication between a contained medicament within the housing of the medicament delivery device and the delivery member 12 can be established.

In one example, the counter fastener is an integral part of the housing of the medicament delivery device. Alternatively, as shown in Fig. 2, the counter fastener 132 can be an independent component that is configured to be attached to the housing of the medicament delivery device before a medicament delivery operation, e.g., via a snap-fit, bayonet engagement or a thread engagement. For example, as shown in Fig. 2, the counter fastener 132 is a part of a retainer 13 comprising a snap-fit arm 131 configured to be attached to the housing of the medicament delivery device. In this example, the retainer 13 can be attached to the housing of the medicament delivery device during manufacture of the medicament delivery device.

In the example, one of the fastener 112 and the counter fastener 132 is a screw thread, and the other one of the fastener 112 and the counter fastener 132 is a thread follower, e.g., a protrusion positioned within the screw thread or another screw thread. For example, as shown in Fig. 2, the fastener 112 is a screw thread and the counter fastener 132 is a screw thread. In this example, the delivery member hub 11 is axially and rotationally movable relative to the housing of the medicament delivery device in the distal direction of the tubular cap 3; 3'; 3" between an unattached position where the fastener 112 is not attached to the counter fastener 132 and an attached position where the fastener 112 is attached to the counter fastener 132.

Alternatively, one of the fastener and the counter fastener is a bayonet track and the other one of the fastener and the counter fastener is a bayonet track follower. In this example, the delivery member hub 11 can be axially immovable and rotationally movable relative to the housing of the medicament delivery device about the direction of the longitudinal axis L. For example, the bayonet track can be a circumferential groove with a cut-out (e.g., for manufacture purposes), and the bayonet follower can be a protrusion positioned within the circumferential groove so that a rotation of the delivery member hub about the longitudinal axis relative to the housing can move the protrusion within from the unattached position (e.g., close to the end with the cut-out) to the attached position (e.g., far away from the end with the cut-out).

Optionally, the delivery member assembly 1 comprises the inner cap 10, the inner cap is releasably attached to the housing of the medicament delivery device via a rotational engagement. In one example, as shown in Figs 2-3, the rotational engagement is a screw thread engagement. In this example, the inner cap 10 comprises a screw thread 102, and the housing of the medicament delivery device comprises a counter screw thread 133. In a preferred example, the counter screw thread 133 extends from an outer surface of the retainer 13. Alternatively, the counter screw thread can be an integral part of the housing of the medicament delivery device.

In one example where the delivery member assembly comprises the inner cap 10, the delivery member hub 11 is arranged within the tubular inner cap 10. The delivery member hub 11 is rotationally immovable and axially movable relative to the inner cap 10 via a first engagement between the delivery member hub 11 and the inner cap 10. In one example, as shown in Fig. 3, the first engagement is formed by a cut-out 113 in the delivery member hub 11, the cut-out 113 is open to a direction transverse to the longitudinal axis, and a longitudinal rib 103 extending from an inner surface of the inner cap 10. It should be noted that the first engagement can also be formed by a longitudinally extending rib on the inner cap and a longitudinally extending rib on the delivery member hub, or the first engagement can be formed by a longitudinally extending rib on the delivery member hub and a cut-out/recess on the inner cap, wherein the cut-out/recess is open to the direction transverse to the longitudinal axis, or the first engagement is formed by a non-circular cross-section body section of the delivery member hub matching with a non-circular cross-section body section of the inner cap.

In one example where the inner cap 10 is releasably attached to the housing of the medicament delivery device via a screw thread engagement and the delivery member hub 11 is also attached to the housing of the medicament delivery device via a screw thread engagement, the thread engagement between the inner cap 10 and the housing of the delivery member hub is a proximally directed thread, and the thread engagement between the delivery member hub 11 and the housing of the medicament delivery device is a distally directed thread. Therefore, when the inner cap 10 rotates relative to the housing of the medicament delivery device, the inner cap 10 can be moved away from the housing of the medicament delivery device and be detached from the housing via the proximally directed thread engagement, in the meantime, the delivery member hub 11 will be rotated by the inner cap 10, via the first engagement, relative to the housing of the medicament delivery device. Thereby the delivery member hub 11 can be moved towards the housing of the delivery member device and be attached to the housing of the delivery member device via the distally directed thread.

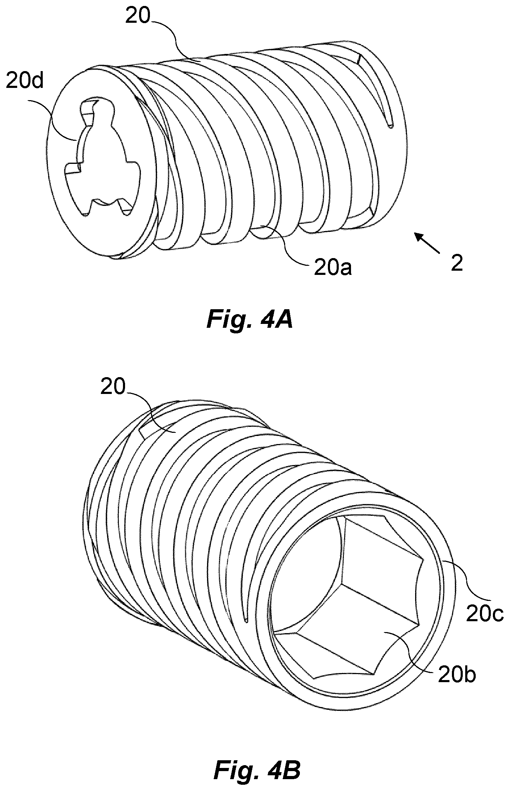

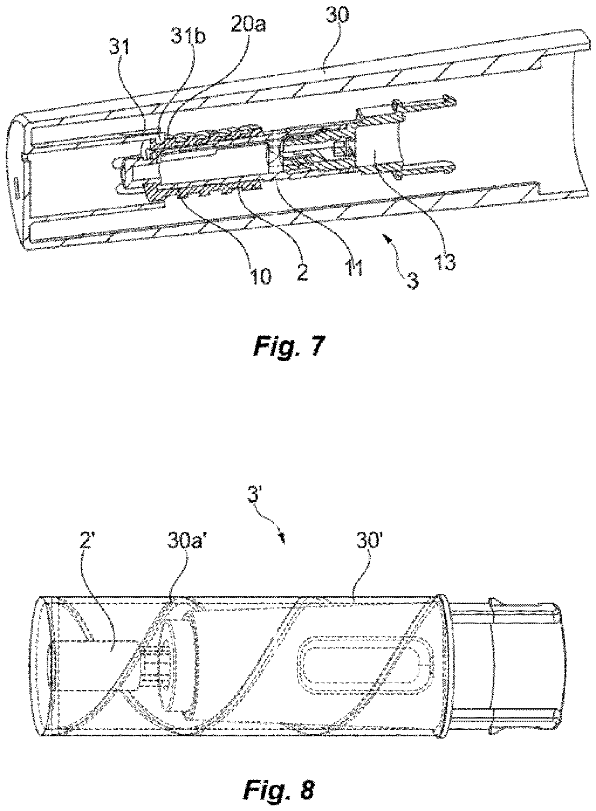

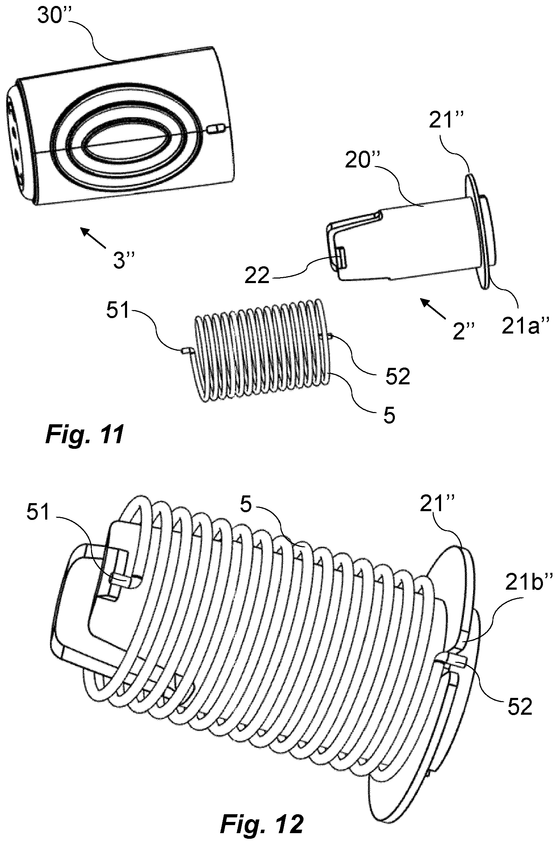

The adapter 2; 2'; 2" comprises a body 20; 20'; 20", as shown in Figs 4A-4B, and Fig. 11, operably connected to delivery member hub 11, so that the adapter 2; 2'; 2" is rotationally immovable and axially movable relative to the delivery member hub 11. The adapter 2; 2'; 2" is arranged within the tubular cap 3; 3'; 3", as shown in Fig. 7-8, 10, and 13-14 and the adapter 2; 2'; 2" comprises a distally directed surface 20c; 21a", as shown in Fig. 4B and Fig.11. In a preferred example, the body 20; 20'; 20" of the adapter 2; 2'; 2" is a sleeve, as shown in Figs 4A-4B. Alternatively, the body of the adapter can be a ring or be semi-circular or can be any suitable shape depending on the design. In a preferred example, the adapter 2; 2'; 2" is configured to be axially movable relative to the tubular cap 3; 3'; 3" within a predetermined axial distance, so that when the user removes the tubular cap 3; 3'; 3" from the medicament delivery device, the adapter 2; 2'; 2" can also be removed with the tubular cap 3; 3'; 3". Examples of the connection between the adapter 2; 2'; 2" and the tubular cap 3; 3'; 3" will be explained in detail later.

In one example where the delivery member assembly comprises the inner cap 10, the inner cap 10 is arranged within the body 20; 20'; 20" of the adapter 2, 2', 2". The inner cap 10 is rotationally immovable relative to the body 20; 20'; 20" of the adapter 2; 2'; 2" via a second engagement between the inner cap 10 and the body 20; 20'; 20" of the adapter 2; 2'; 2". In a preferred example, the second engagement is formed by a non-circular cross-section body 20b of the body of the adapter 2; 2'; 2" matching with a non-circular cross-section body 101 of the inner cap 10, as shown in Fig. 3 and Fig. 4B. Alternatively, the second engagement is formed by a longitudinally extending rib on the inner cap and a longitudinally extending rib on the body of the adapter; or the second engagement is formed by a longitudinally extending rib on the inner cap and a cut-out/recess on the body of the adapter, and the cut-out/recess is open to the direction transverse to the longitudinal axis; or the first engagement is formed by a longitudinally extending rib on the body of the adapter and a cut-out/recess on the inner cap, and the cut-out/recess is open to the direction transverse to the longitudinal axis.

It should be noted that when a non-circular cross-section body of one component is used to match with another non-circular cross-section body of the other component, a rotational engagement is formed, meaning that the two components now can only rotate together. For example, the non-circular cross-section body can be a polygonal cross-section body, star shaped cross-section body, or heart shaped cross-section body.

In this example, preferably, the adapter 2;2';2" is axially attached to the inner cap 10. For example, the inner cap 10 comprises a proximal neck portion 104, as shown in Fig. 2, and the adapter 2 comprises a ledge 20d extending in the direction transverse to the longitudinal axis L from the inner surface of the body 20 of the adapter 2, as shown in Fig. 4A. The ledge 20d engages with the neck portion 104 of the inner cap 10, as shown in Fig. 5, so that when the user removes the adapter 2 from the medicament delivery device, the inner cap 10 is removed together with the adapter 2.

In another example, the adapter is engaged with the delivery member hub directly via an engagement. The engagement in this example can be formed by: a longitudinally extending rib on the delivery member hub and a longitudinally extending rib on the body of the adapter; or the engagement is formed by a longitudinally extending rib on the delivery member hub and a cut-out/recess on the body of the adapter, wherein the cut-out/recess is open to the direction transverse to the longitudinal axis; or the engagement is formed by a longitudinally extending rib on the body of the adapter and a cut-out/recess on the delivery member hub, wherein the cut-out/recess is open to the direction transverse to the longitudinal axis; or the engagement is formed by a non-circular cross-section body section of the body of the adapter matching with a non-circular cross-section body section of the delivery member hub.

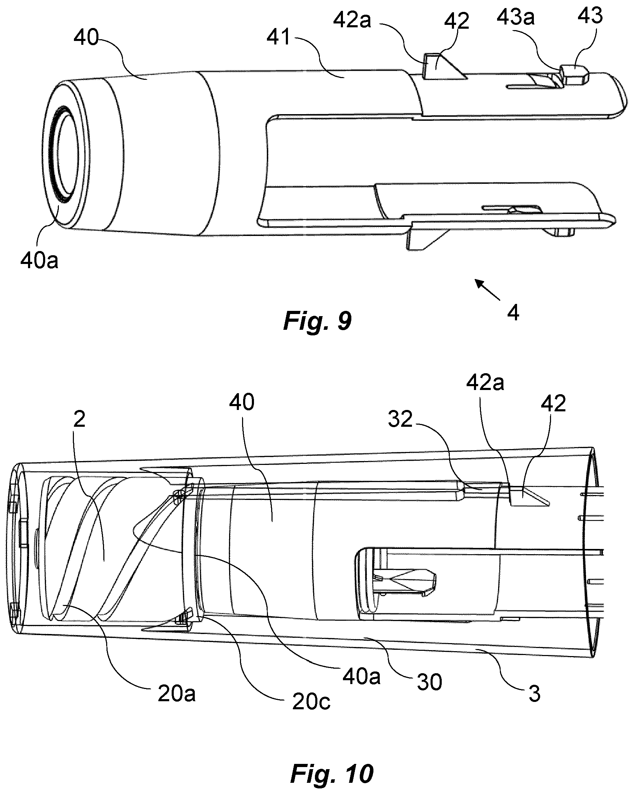

The delivery member guard 4 is axially movable relative to the tubular cap 3; 3'; 3" from a distal position to a proximal position. In a preferred example, the delivery member guard 4 comprises a tubular body 40 configured to surround the delivery member 12. In a preferred example, the delivery member guard 4 comprises a proximally directed surface 40a adjacent to the distally directed surface of the adapter 20c; 21a", as shown in Fig. 10. The proximal end of the tubular body 40 defines the proximally directed surface 40a. Alternatively, the delivery member guard comprises a proximally directed arm, and the proximal end of the arm defines the proximally directed surface. In another example, the delivery member guard 4 comprises a proximally directed surface 42a adjacent to a distally directed surface 32 of the tubular cap 3, as shown in Fig. 10. In a preferred example, the delivery member guard 4 comprises a distally extended arm 41. The distally extended arm 41 can comprise one or more protrusions 42, 43 for multiple design needs, e.g., for interacting with a powerpack to triggering a medicament delivery operation and/or for interacting with a lock, so that the delivery member guard can be locked in the proximal position after use. For example, the proximally directed surface 42a can be defined by one protrusion 42.

It should be noted that Fig. 10 is drawn to illustrate these two examples with the same figure. However, preferably, the delivery member guard 4 will only press on either the adapter 2 or the tubular cap 3, as the axial movement of the delivery guard 4 is configured to create a relative axial movement between the adapter 2 and the tubular cap 3. If the delivery member guard presses on both the adapter 2 and the tubular cap 3, a force conditioning design needs to be arranged. For example, a damper or a brake can be added either between the delivery member guard and the tubular cap or between the delivery member guard and the adapter, so that the relative axial movement between the adapter 2 and the tubular cap 3 can be created.

The sub-assembly of the invention further comprises a rotation mechanism arranged between the tubular cap 3; 3'; 3' and the adapter 2; 2'; 2". The rotation mechanism is configured so that a rotation of the adapter 2; 2'; 2" about the longitudinal axis L relative to the tubular cap 3; 3'; 3" is triggered when the delivery member guard 4 moves from the distal position to the proximal position, so that the delivery member hub 11 is moved from the proximal position to the distal position by the rotation of the adapter 2; 2'; 2".

In one example where one of the fastener 112 of the delivery member hub 11 and the counter fastener 132 of the housing of the medicament delivery device is the screw thread, and the other one of the fastener 112 and the counter fastener 132 is the thread follower, the rotation direction of the adapter 2; 2'; 2" is dependent on the screw thread direction that can lead the delivery member hub 11 to move in the distal direction relative to the tubular cap 3; 3'; 3". For example, if a clockwise rotation of the delivery member hub 11 (when viewing from the proximal end of the delivery member hub) causes the delivery member hub 11 to move, along the screw thread, in the distal direction relative to the tubular cap 3; 3'; 3", the rotation mechanism is configured to rotate the adapter 2; 2'; 2" in the clockwise direction.

As the adapter 2; 2'; 2" is operably connected to the delivery member hub 11, the rotation of the adapter 2; 2'; 2" relative to the tubular cap 3; 3'; 3" rotates the delivery member hub 11 relative to the tubular cap 3; 3'; 3". As the tubular cap 3; 3'; 3" stays immovable relative to the housing of the medicament delivery device, the rotation of the delivery member hub 11 relative to the tubular cap 3; 3'; 3" moves the delivery member hub 11 from the proximal position where the fastener 112 is not attached to the counter fastener 132 and the distal position where the fastener 112 is attached to the counter fastener 132. Therefore, the delivery member hub 11 and the delivery member 12 are attached to the housing of the medicament delivery device as mentioned above.

In one example, the rotation mechanism comprises a screw thread 20a; 30a' and a protrusion 31b positioned within the screw thread 20a; 30a'. One of the screw thread 20a; 30a' and the protrusion 31b extends from the body 20; 20' of the adapter 2; 2'; and the other one of the screw thread 20a; 30a' and the protrusion 31b extends from the body 30; 30' of the tubular cap 3; 3' towards the body 20; 20' of the adapter 2; 2'.

In one example, the screw thread 20a is arranged on the outer surface of the adapter 2, as shown in Fig. 4A and Fig. 10. In this example, the protrusion 31b extends from an inner surface of the body 30 of the tubular cap 3 towards the body 20 of the adapter 2, as shown in Fig. 6-7 and Fig. 10. For example, the tubular cap 3 comprises a distally extending arm 31 that extends along the inner surface of the body 30. The distally extending arm 31 comprises an arm body 31a, and the protrusion 31b is extending from the arm body 31a. Alternatively, the protrusion 31b can extend directly from the body 30. In another example, the screw thread 30a' is arranged on the inner surface of the body 30' of the tubular cap 3', as shown in Fig. 8. In this example, the protrusion (not shown) extends from an outer surface of the body 20' of the adapter 2' towards the body 30' of the tubular cap 3'. When the delivery member guard 4 moves from the distal position to the proximal position, the delivery member guard 4 moves the adapter 2; 2' in the proximal direction relative to the tubular cap 3; 3' following the screw thread. Thereby, the adapter 2; 2' rotates relative to the tubular cap 3; 3'. Alternatively, in one example where the delivery member guard 4 is engaged with the tubular cap 3; 3' as mentioned above, the delivery member guard 4 can either move the tubular cap 3; 3' relative to the adapter 2; 2' to cause the adapter 2; 2' to rotate, or the delivery member guard 4 can facilitate the user who is required to pull the tubular cap 3; 3' relative to the adapter 2;2' to cause the adapter 2; 2' to rotate.

In one example where the adapter 2; 2' is configured to be axially movable relative to the tubular cap 3; 3' within the predetermined axial distance, wherein the predetermined axial distance can be defined by the thread and the protrusion between the adapter 2; 2' and the tubular cap 3; 3'.

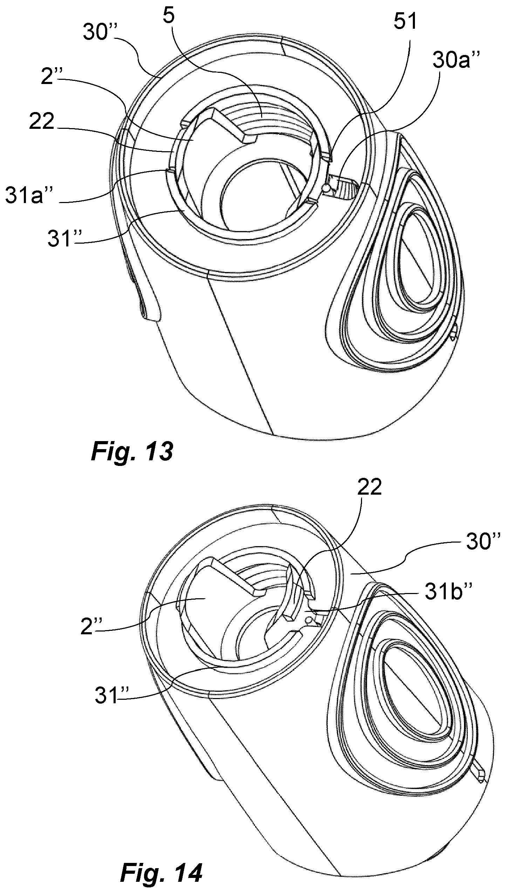

In another example, as shown in Figs 11-14, the rotation mechanism comprises a torsion spring 5 winding around the longitudinal axis L. The torsion spring 5 extends between a first end 51 and a second end 52. The first end 51 is fixed to the tubular cap 3". In one example, a cut-out/recess 30a" is in the wall of the body 30" of the tubular cap 3", and the first end 51 of the torsion spring is positioned into the cut-out/recess 30a", as shown in Fig. 14. The second end 52 is fixed to the body 20" of the adapter 2". In one example, a cut-out/recess 21b" is in the wall of the body 20" of the adapter 2", and the second end 52 of the torsion spring 5 is positioned within the cut-out/recess 21b", as shown in Fig. 12. The adapter 2" comprises an engaging member 22 extending from the body 20" of the adapter 2". The tubular cap 3" comprises a counter engaging member 31b" aligned with the engaging member 22 of the adapter 2" in a circumferential direction when the delivery member guard 4 is in the distal position, as shown in Fig. 13, and offset relative to the engaging member 22 of the adapter 2" in the circumferential direction when the delivery member guard 4 is in the proximal position, as shown in Fig. 14. Therefore, when the engaging member 22 is offset relative to the counter engaging member 31b", the torsion spring 5 rotates the adapter 2" relative to the tubular cap 3".

In one example, the engaging member 22 is a rib extending in the direction transverse to the longitudinal axis L from the outer surface of the body 20" of the adapter 2", as shown in Figs 11-12. In this example, the counter engaging member 31b' is a cut-out in the wall of the body 30" of the tubular cap 3". Alternatively, the engaging member can be a cut-out/recess in the wall of the body of the adapter, and the counter engaging member can be a rib extending from the inner surface of the body of the tubular cap.

In one example, the adapter 2" comprises a flange 21" extending from the body 20" of the adapter 2". The flange 21" defines the distally directed surface 21a', as shown in Fig. 12. Alternatively, the distal end of the body 20 of the adapter 2; 2' defines the distally directed surface 21a'. In one example where the adapter 2" comprises the flange 21", the cut-out 21b" is arranged in the flange 21".

In one example where the adapter 2" is configured to be axially movable relative to the tubular cap 3" within the predetermined axial distance, the predetermined axial distance can be defined by a snap-fit connection between the tubular cap 3" and the adapter 2". For example, the snap-fit connection can be formed by an annular ledge on the inner surface of body 30" of the tubular cap 3" and a hook on the body 20" of the adapter 2". The hook comprises a distally directed surface configured to engage with the annular ledge when the delivery member guard 4 is in the proximal position.

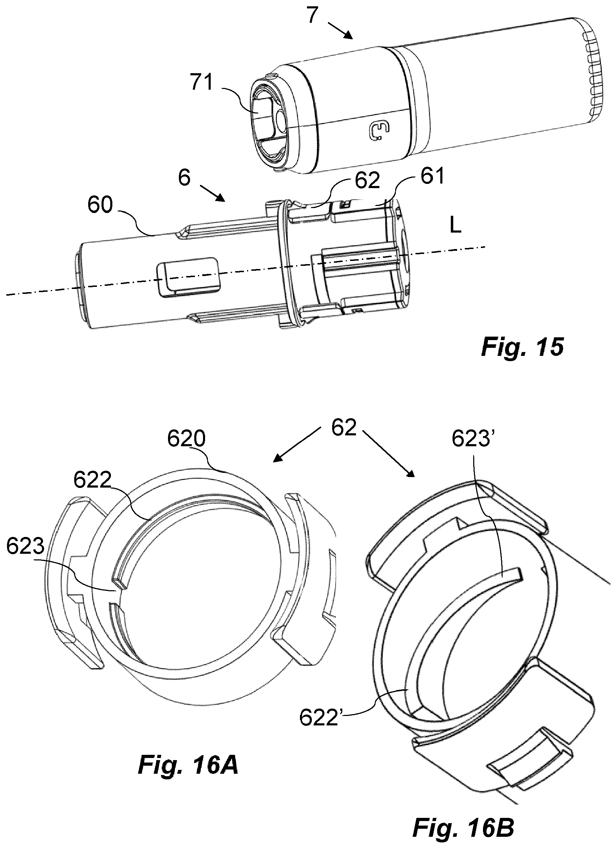

The invention further provides a front sub-assembly 6 for a medicament delivery device, as shown in Fig. 15. In a preferred example, the front sub-assembly 6 is configured to be used in a cassette of a medicament delivery device. The medicament delivery device with the cassette usually is formed by the disposable cassette and a reusable drive unit 7. The cassette unit 6 comprises a medicament container. The cassette 6 is configured to be attached to the drive unit 7 before the operation of the medicament delivery.

The front sub-assembly comprisesthe sub-assembly as defined above. The front sub-assembly further comprises a body 60 extending along the longitudinal axis L from a proximal end to a distal end. The delivery member guard 4 is coaxially attached to the body 60 and is axially movable relative to the body 60 along the longitudinal axis L. In a preferred example, the body 60 is the body of the cassette and is configured to accommodate a medicament container. In this example, the body 60 is configured to be attached to the drive unit 7.

In a preferred example, the front sub-assembly comprises a lock member 62 attached to the body 20. The lock member 62 is movable relative to the delivery member guard 4 between a locked position and an unlocked position. As shown in Figs 16A-16B, the lock member 62 comprises a distally directed surface 622; 622' adjacent to a second proximally directed surface 43a of the delivery member guard 4 in the locked position; and the distally directed surface of the lock member 62 is spaced apart in a circumferential direction relative to the longitudinal axis L from the proximally directed surface of the delivery member guard 4 in the unlocked position. For example, a cut-out 623 is aligned with the second proximally directed surface 43a of the delivery member guard 4 when the lock member 62 is in the unlocked position. Alternatively, the second proximally directed surface 43a of the delivery member guard 4 is aligned with the distally directed surface 622' that extends beyond a second distally directed surface 623' in the distal direction of the body 60 in the locked position; and the delivery member guard 4 is aligned with the second distally directed surface 623' in the unlocked position.

In a preferred example, the lock member 62 is rotatable relative to the delivery member guard 4 around the longitudinal axis L between the locked position and the unlocked position. Alternatively, the lock member is a pin slidable relative to the delivery member guard in the direction transverse to the longitudinal axis between a locked position and an unlocked position. In a preferred example, the lock member is configured to be removed from the delivery member guard and the body in the unlocked position.

Therefore, when the lock member 62 is in the unlocked position, the delivery member guard 4 can move from the proximal position to the distal position and thus trigger the rotation mechanism of the sub-assembly as mentioned above, thereby the delivery member hub and the delivery member can be attached to the body 60 (in the example, the body 60 is the housing of the medicament delivery device), as mentioned above.

In one example, the front sub-assembly comprises a biasing member arranged between a distally directed surface of the body 60 and a proximally directed surface of the delivery member guard 4 at the other end, so that when the lock member 62 is in the unlocked position, the delivery member guard 4 is moved by the biasing member from the distal position to the proximal position. In another example, the delivery member guard 4 is moved from the distal position to the proximal position by the drive unit 7. For example, the drive unit comprises an arm extending from a wall of the drive unit towards the longitudinal axis L. When the body is attached to the drive unit, the arm pushes on the distal end of the delivery member guard, which moves the delivery member guard from the distal position to the proximal position.

Furthermore, the invention provides a method of attaching a delivery member to a medicament delivery device. The method comprises the steps of: providing a tubular cap 3; 3';3" extending along a longitudinal axis L, wherein the tubular cap 3; 3'; 3" houses an adapter 2; 2'; 2"; providing a delivery member hub 11, a delivery member 12 fixed to the delivery member hub 11, and a delivery member guard 4 in contact with the adapter 2; 2'; 2" in the direction of the longitudinal axis L; and subsequently triggering a rotation of the adapter 2; 2'; 2" relative to the tubular cap 3; 3'; 3" about the longitudinal axis L by using the delivery member guard 4 to axially move the adapter 2; 2'; 2" in the direction of the longitudinal axis L towards the tubular cap 3; 3'; 3", thereby rotating the delivery member hub 11 with the adapter 2; 2'; 2" relative to the tubular cap 3; 3'; 3", and thereby attaching the delivery member 12 to the medicament delivery device by the rotation of the delivery member hub 11.

Preferably, the step of triggering the rotation of the adapter 2; 2'; 2" relative to the tubular cap 3; 3'; 3" about the longitudinal axis L by using the delivery member guard 4 to axially move the adapter 2; 2'; 2" in the direction of the longitudinal axis L comprises the steps of: using the delivery member guard 4 to axially move the adapter 2; 2'; 2" out from a rotational block structure of the tubular cap 3; 3'; 3"; and rotating the adapter 2; 2'; 2" using a torsion spring 5 that is fixed between the tubular cap 3; 3'; 3" and the adapter 2; 2'; 2".

Preferably, the step of triggering the rotation of the adapter 2; 2' relative to the tubular cap 3; 3' about the longitudinal axis L by using the delivery member guard 4 to axially move the adapter 2; 2' in the direction of the longitudinal axis L comprises the steps of: rotating the adapter 2; 2' using a converter assembly that is configured to convert the axial movement of the adapter into a rotational movement of the adapter 2; 2'. In a preferred example, the converter assembly is formed by the thread 20a, 30a and the protrusion 31b positioned in the thread. One of the thread and the protrusion is positioned on the outer surface of the adapter 2; 2' and the other one of the thread and the protrusion is positioned on the inner surface of the tubular cap 3; 3'. In a preferred example, the delivery member is attached to the medicament delivery device via the thread or bayonet connection between the delivery member hub and the medicament delivery device.

The inventive concept has mainly been described above with reference to a few examples. However, as is readily appreciated by a person skilled in the art, other embodiments than the ones disclosed above are equally possible within the scope of the inventive concept, as defined by the appended claims.

Some aspects of the invention are described in the clauses below. below, which are only insofar a part of the invention, as they correspond to the subject-matter defined in the claims.
1. A sub-assembly of a medicament delivery device, the sub-assembly comprising:
   a tubular cap, an adapter, a delivery member assembly, and a delivery member guard;
   wherein the tubular cap extends along a longitudinal axis between a proximal end and a distal end;
   wherein the delivery member assembly comprises a delivery member hub, and a delivery member attached to the delivery member hub; wherein the delivery member hub comprises a fastener configured to be attached to a counter fastener of a housing of the medicament delivery device via a relative rotation between the
   fastener and the counter fastener; wherein the delivery member hub is rotatable relative to the housing of the medicament delivery device about the longitudinal axis between an unattached position where the fastener is not attached to the counter fastener and an attached position where the fastener is attached to the counter fastener;
   wherein the adapter comprises a body operably connected to the delivery member hub, so that the adapter is rotationally immovable and axially movable relative to the delivery member hub; wherein the adapter is arranged within the tubular cap;
   wherein the adapter comprises a distally directed surface;
   wherein the delivery member guard comprises a proximally directed surface adjacent to the distally directed surface of the adapter; wherein the delivery member guard is axially movable relative to the tubular cap from a distal position to a proximal position;
   wherein a rotation mechanism is arranged between the tubular cap and the adapter;
   and wherein the rotation mechanism is configured so that a rotation of the adapter about the longitudinal axis relative to the tubular cap is triggered when the delivery member guard moves from the distal position to the proximal position, so that the delivery member hub is moved from the unattached position to the attached position by the rotation of the adapter.
2. The sub-assembly according to clause 1, wherein the rotation mechanism comprises a screw thread and a protrusion positioned within the screw thread; wherein one of the screw thread and the protrusion extends from the body of the adapter; and wherein the other one of the screw thread and the protrusion extends from a body of the tubular cap towards the body of the adapter.
3. The sub-assembly according to clause 2, wherein the screw thread is arranged on an outer surface of the body of the adapter; wherein the tubular cap comprises a distally extending arm extending from the body of the tubular cap in the direction of the longitudinal axis; and wherein the protrusion extends in a direction transverse to the longitudinal axis from the distally extending arm.
4. The sub-assembly according to clause 2, wherein the screw thread is arranged on an inner surface of the body of the tubular cap; and wherein the protrusion extends in a direction transverse to the longitudinal axis from an outer surface of the body of the adapter.
5. The sub-assembly according to clause 1, wherein the rotation mechanism comprises a torsion spring winding around the longitudinal axis; wherein the torsion spring extends between a first end and a second end; wherein the first end is fixed to the tubular cap; wherein the second end is fixed to the body of the adapter; wherein the adapter comprises an engaging member extending from the body of the adapter; and wherein the tubular cap comprises a counter engaging member aligned with the engaging member of the adapter in a circumferential direction when the delivery member guard is in the distal position, and offset relative to the engaging member of the adapter in the circumferential direction when the delivery member guard is in the proximal position.
6. The sub-assembly according to clause 5, wherein the engaging member is a rib extending in the direction transverse to the longitudinal axis from an outer surface of the body of the adapter; wherein the counter engaging member is a cut-out in a wall of the body of the tubular cap.
7. The sub-assembly according to clause 5 or 6, wherein the adapter comprises a flange extending from the body of the adapter; wherein the flange defines the distally directed surface; wherein a cut-out is arranged in the flange and wherein the second end of the torsion spring is attached within the cut-out of the flange.
8. The sub-assembly according to any of the preceding clauses, wherein the delivery member assembly comprises a tubular inner cap; wherein the delivery member hub is arranged within the tubular inner cap; wherein the delivery member hub is rotationally immovable and axially movable relative to the inner cap via a first engagement between the delivery member hub and the inner cap; wherein the inner cap is arranged within the body; and wherein the inner cap is rotationally immovable relative to the body of the adapter via a second engagement between the inner cap and the body of the adapter.
9. The sub-assembly according to clause 8, wherein the first engagement is formed by a longitudinally extending rib on the inner cap and a longitudinally extending rib on the delivery member hub; or the first engagement is formed by a longitudinally extending rib on the inner cap and a cut-out/recess on the delivery member hub, wherein the cut-out/recess is open to the direction transverse to the longitudinal axis; or the first engagement is formed by a longitudinally extending rib on the delivery member hub and a cut-out/recess on the inner cap, wherein the cut-out/recess is open to the direction transverse to the longitudinal axis; or the first engagement is formed by a non-circular cross-section body section of the delivery member hub matching with a non-circular cross-section body section of the inner cap.
10. The sub-assembly according to clause 8 or 9, wherein the second engagement is formed by a longitudinally extending rib on the inner cap and a longitudinally extending rib on the body of the adapter; or the second engagement is formed by a longitudinally extending rib on the inner cap and a cut-out/recess on the body of the adapter, wherein the cut-out/recess is open to the direction transverse to the longitudinal axis; or the second engagement is formed by a longitudinally extending rib on the body of the adapter and a cut-out/recess on the inner cap, wherein the cut-out/recess is open to the direction transverse to the longitudinal axis; or the second engagement is formed by a non-circular cross-section body section of the body of the adapter matching with a non-circular cross-section body section of the inner cap.
11. The sub-assembly according to any of the clauses 1-7, wherein the adapter is directly engaged with the delivery member hub via an engagement formed by: a longitudinally extending rib on the delivery member hub and a longitudinally extending rib on the body of the adapter; or the engagement is formed by a longitudinally extending rib on the delivery member hub and a cut-out/recess on the body of the adapter, wherein the cut-out/recess is open to the direction transverse to the longitudinal axis; or the engagement is formed by a longitudinally extending rib on the body of the adapter and a cut-out/recess on the delivery member hub, wherein the cut-out/recess is open to the direction transverse to the longitudinal axis; or the engagement is formed by a non-circular cross-section body section of the body of the adapter matching with a non-circular cross-section body section of the delivery member hub.
12. The sub-assembly according to any of the preceding clauses, wherein one of the fastener and the counter fastener is a screw thread, and the other one of the fastener and the counter fastener is a screw thread follower; and wherein the delivery member hub is axially movable relative to the housing of the medicament delivery device in the distal direction of the tubular cap.
13. The sub-assembly according to any of the preceding clauses, wherein one of the fastener and the counter fastener is a bayonet track and the other one of the fastener and the counter fastener is a bayonet track follower.
14. The sub-assembly according to any of the preceding clauses, wherein the delivery member guard comprises a tubular body configured to surround the delivery member; and wherein the proximal end of the tubular body defines the proximally directed surface.
15. A front sub-assembly for a medicament delivery device, the front sub-assembly comprising the sub-assembly according any of the preceding clauses; wherein the front sub-assembly further comprises:
   a body extending along the longitudinal axis from a proximal end to a distal end;
   the delivery member guard coaxially attached to the body and being axially movable relative to the body along the longitudinal axis; and
   a lock member attached to the body;
   wherein the lock member is movable relative to the delivery member guard between a locked position and an unlocked position; and
   wherein the lock member comprises a distally directed surface adjacent to a second proximally directed surface of the delivery member guard in the locked position; and
   wherein the distally directed surface of the lock member is spaced apart in a circumferential direction relative to the longitudinal axis from the proximally directed surface of the delivery member guard in the unlocked position.
16. The front sub-assembly according to clause 15, wherein the lock member is rotatable relative to the delivery member guard around the longitudinal axis between the locked position and the unlocked position.
17. The front sub-assembly according to clause 15 or 16 comprising a biasing member arranged between a distally directed surface of the delivery member guard and a proximally directed surface of the body.
18. A medicament delivery device comprising the front sub-assembly according to any of clauses 15-17.
19. A method of attaching a delivery member to a medicament delivery device, the method comprising the following steps in the following order:
   providing:
      a tubular cap extending along a longitudinal axis; wherein the tubular cap houses an adapter;
      a delivery member hub;
      a delivery member fixed to the delivery member hub;
      a delivery member guard in contact with the adapter in the direction of the longitudinal axis;
   triggering a rotation of the adapter relative to the tubular cap about the longitudinal axis by using the delivery member guard to axially move the adapter in the direction of the longitudinal axis towards the tubular cap, thereby rotating the delivery member hub with the adapter relative to the tubular cap, and thereby attaching the delivery member to the medicament delivery device by the rotation of the delivery member hub.
20. The method according to clause 19, wherein the step of triggering a rotation of the adapter relative to the tubular cap about the longitudinal axis by using the delivery member guard to axially move the adapter in the direction of the longitudinal axis comprising the steps of:
   using the delivery member guard to axially move the adapter out from a rotational block structure of the tubular cap; and
   rotating the adapter using a torsion spring that is fixed between the tubular cap and the adapter.
21. The method according to clause 19, wherein the step of triggering a rotation of the adapter relative to the tubular cap about the longitudinal axis by using the delivery member guard to axially move the adapter in the direction of the longitudinal axis comprising the steps of:
   rotating the adapter using a converter assembly that is configured to convert the axial movement of the adapter into a rotational movement of the adapter.
22. The method according to clause 21, wherein the converter assembly is formed by a thread and a protrusion positioned in the thread; and wherein one of the thread and the protrusion is positioned on an outer surface of the adapter and the other one of the thread and the protrusion is positioned on an inner surface of the tubular cap.
23. The method according to any of clauses 19-22, wherein:
   the delivery member is attached to the medicament delivery device via a thread or
   bayonet connection between the delivery member hub and the medicament delivery device.

## Claims

1. A sub-assembly of a medicament delivery device, the sub-assembly comprising:
a tubular cap (3; 3'; 3"), an adapter (2; 2'; 2"), a delivery member assembly (1), and
a delivery member guard (4);
wherein the tubular cap (3; 3'; 3") extends along a longitudinal axis (L) between a proximal end and a distal end;
wherein the delivery member assembly (1) comprises a delivery member hub (11), and a delivery member (12) attached to the delivery member hub (11); wherein the delivery member hub (11) comprises a fastener (112) configured to be attached to a counter fastener (132) of a housing of the medicament delivery device via a relative rotation between the fastener (112) and the counter fastener (132); wherein the delivery member hub (11) is rotatable relative to the housing of the medicament delivery device about the longitudinal axis (L) between an unattached position where the fastener (112) is not attached to the counter fastener (132) and an attached position where the fastener (112) is attached to the counter fastener (132);
wherein the adapter (2; 2'; 2") comprises a body (20; 20'; 20") operably connected to the delivery member hub (11), so that the adapter (2; 2'; 2") is rotationally immovable and axially movable relative to the delivery member hub (11); wherein
the adapter (2; 2'; 2") is arranged within the tubular cap (3; 3'; 3"); wherein the adapter comprises a distally directed surface (20c; 21a");
wherein the delivery member guard (4) comprises a proximally directed surface (40a) adjacent to the distally directed surface (20c; 21a") of the adapter (2; 2'; 2");
wherein the delivery member guard (4) is axially movable relative to the tubular cap (3; 3'; 3") from a distal position to a proximal position;
wherein a rotation mechanism is arranged between the tubular cap (3; 3'; 3") and the adapter (2; 2'; 2"); and wherein the rotation mechanism is configured so that a rotation of the adapter (2; 2'; 2") about the longitudinal axis (L) relative to the tubular cap (3; 3'; 3") is triggered when the delivery member guard (4) moves from the distal position to the proximal position, so that the delivery member hub (11) is moved from the unattached position to the attached position by the rotation of the adapter (2; 2'; 2").

2. The sub-assembly according to claim 1, wherein the rotation mechanism comprises a screw thread (20a; 30a') and a protrusion (31b) positioned within the screw thread (20a; 30a'); wherein one of the screw thread (20a; 30a') and the protrusion (31b) extends from the body (20; 20') of the adapter (2; 2'); and wherein the other one of the screw thread (20a; 30a') and the protrusion (31b) extends from a body (30; 30') of the tubular cap (3; 3') towards the body (20; 20') of the adapter (2; 2').

3. The sub-assembly according to claim 2, wherein the screw thread (20a) is arranged on an outer surface of the body (20) of the adapter (2); wherein the tubular cap (3) comprises a distally extending arm (31a) extending from the body (30) of the tubular cap in the direction of the longitudinal axis (L); and wherein the protrusion (31b) extends in a direction transverse to the longitudinal axis (L) from the distally extending arm (31a).

4. The sub-assembly according to claim 2, wherein the screw thread (30a') is arranged on an inner surface of the body (30') of the tubular cap (3'); and wherein the protrusion extends in a direction transverse to the longitudinal axis (L) from an outer surface of the body (20') of the adapter (2').

5. The sub-assembly according to claim 1, wherein the rotation mechanism comprises a torsion spring (5) winding around the longitudinal axis (L); wherein the torsion spring (5) extends between a first end (51) and a second end (52); wherein the first end (51) is fixed to the tubular cap (3"); wherein the second end (52) is fixed to the body (20") of the adapter (2'); wherein the adapter (2") comprises an engaging member (22) extending from the body (20") of the adapter (2"); and wherein the tubular cap (3") comprises a counter engaging member (31b") aligned with the engaging member (22) of the adapter (2") in a circumferential direction when the delivery member guard (4) is in the distal position, and offset relative to the engaging member (22) of the adapter (2") in the circumferential direction when the delivery member guard (4) is in the proximal position.

6. The sub-assembly according to claim 5, wherein the engaging member (22) is a rib extending in the direction transverse to the longitudinal axis (L) from an outer surface of the body (20") of the adapter (2"); wherein the counter engaging member (31b') is a cut-out in a wall of the body (30") of the tubular cap (3").

7. The sub-assembly according to claim 5 or 6, wherein the adapter comprises a flange (21") extending from the body (20") of the adapter; wherein the flange (21") defines the distally directed surface (21a'); wherein a cut-out (21b") is arranged in the flange (21") and wherein the second end (52) of the torsion spring (5) is attached within the cut-out (21b") of the flange (21").

8. The sub-assembly according to any of the preceding claims, wherein the delivery member assembly (1) comprises a tubular inner cap (10); wherein the delivery member hub (11) is arranged within the tubular inner cap (10); wherein the delivery member hub (11) is rotationally immovable and axially movable relative to the inner cap (10) via a first engagement between the delivery member hub (11) and the inner cap (10); wherein the inner cap (10) is arranged within the body (20; 20'; 20"); and wherein the inner cap (10) is rotationally immovable relative to the body (20; 20'; 20") of the adapter (2; 2'; 2") via a second engagement between the inner cap (10) and the body (20; 20'; 20") of the adapter (2; 2'; 2").

9. The sub-assembly according to claim 8, wherein the first engagement is formed by a longitudinally extending rib on the inner cap and a longitudinally extending rib on the delivery member hub; or the first engagement is formed by a longitudinally extending rib on the inner cap and a cut-out/recess on the delivery member hub, wherein the cut-out/recess is open to the direction transverse to the longitudinal axis; or the first engagement is formed by a longitudinally extending rib on the delivery member hub and a cut-out/recess on the inner cap, wherein the cut-out/recess is open to the direction transverse to the longitudinal axis; or the first engagement is formed by a non-circular cross-section body section of the delivery member hub matching with a non-circular cross-section body section of the inner cap.

10. The sub-assembly according to claim 8 or 9, wherein the second engagement is formed by a longitudinally extending rib on the inner cap and a longitudinally extending rib on the body of the adapter; or the second engagement is formed by a longitudinally extending rib on the inner cap and a cut-out/recess on the body of the adapter, wherein the cut-out/recess is open to the direction transverse to the longitudinal axis; or the second engagement is formed by a longitudinally extending rib on the body of the adapter and a cut-out/recess on the inner cap, wherein the cut-out/recess is open to the direction transverse to the longitudinal axis; or the second engagement is formed by a non-circular cross-section body section of the body of the adapter matching with a non-circular cross-section body section of the inner cap.

11. The sub-assembly according to any of the claims 1-7, wherein the adapter is directly engaged with the delivery member hub via an engagement formed by: a longitudinally extending rib on the delivery member hub and a longitudinally extending rib on the body of the adapter; or the engagement is formed by a longitudinally extending rib on the delivery member hub and a cut-out/recess on the body of the adapter, wherein the cut-out/recess is open to the direction transverse to the longitudinal axis; or the engagement is formed by a longitudinally extending rib on the body of the adapter and a cut-out/recess on the delivery member hub, wherein the cut-out/recess is open to the direction transverse to the longitudinal axis; or the engagement is formed by a non-circular cross-section body section of the body of the adapter matching with a non-circular cross-section body section of the delivery member hub.

12. The sub-assembly according to any of the preceding claims, wherein one of the fastener and the counter fastener is a screw thread, and the other one of the fastener and the counter fastener is a screw thread follower; and wherein the delivery member hub is axially movable relative to the housing of the medicament delivery device in the distal direction of the tubular cap.

13. The sub-assembly according to any of the preceding claims, wherein the delivery member guard (4) comprises a tubular body (40) configured to surround the delivery member (12); and wherein the proximal end of the tubular body (40) defines the proximally directed surface (40a).

14. A front sub-assembly for a medicament delivery device, the front sub-assembly comprising the sub-assembly according any of the preceding claims; wherein the front sub-assembly further comprises:
a body (60) extending along the longitudinal axis (L) from a proximal end to a distal end;
the delivery member guard (4) coaxially attached to the body (60) and being axially movable relative to the body (60) along the longitudinal axis (L); and
a lock member (62) attached to the body (20);
wherein the lock member (62) is movable relative to the delivery member guard (4) between a locked position and an unlocked position; and
wherein the lock member (62) comprises a distally directed surface adjacent to a second proximally directed surface (43a) of the delivery member guard (4) in the locked position; and wherein the distally directed surface of the lock member (62) is spaced apart in a circumferential direction relative to the longitudinal axis (L) from the proximally directed surface of the delivery member guard (4) in the unlocked position.

15. The front sub-assembly according to claim 15, wherein the lock member (62) is rotatable relative to the delivery member guard (4) around the longitudinal axis (L) between the locked position and the unlocked position.

## Patentansprüche

1. Unteranordnung einer Medikamentenabgabevorrichtung, die Unteranordnung umfassend:
eine rohrförmige Kappe (3; 3'; 3"), einen Adapter (2; 2'; 2"), eine Abgabeelementanordnung (1) und einen Abgabeelementschutz (4);
wobei sich die rohrförmige Kappe (3; 3'; 3") entlang einer Längsachse (L) zwischen einem proximalen Ende und einem distalen Ende erstreckt;
wobei die Abgabeelementanordnung (1) eine Abgabeelementnabe (11) und ein Abgabeelement (12), das an der Abgabeelementnabe (11) angebracht ist, umfasst; wobei die Abgabeelementnabe (11) ein Befestigungsmittel (112) umfasst, das konfiguriert ist, um an einem Gegenbefestigungsmittel (132) eines Gehäuses der Medikamentenabgabevorrichtung über eine Relativdrehung zwischen dem Befestigungsmittel (112) und dem Gegenbefestigungsmittel (132) angebracht zu werden; wobei die Abgabeelementnabe (11) zwischen einer nicht angebrachten Position, wo das Befestigungsmittel (112) nicht an dem Gegenbefestigungsmittel (132) angebracht ist, und einer angebrachten Position, wo das Befestigungsmittel (112) an dem Gegenbefestigungsmittel (132) angebracht ist, relativ zu dem Gehäuse der Medikamentenabgabevorrichtung um die Längsachse (L) herum drehbar ist;
wobei der Adapter (2; 2'; 2") einen Körper (20; 20'; 20") umfasst, der mit der Abgabeelementnabe (11) wirkverbunden ist, sodass der Adapter (2; 2'; 2") relativ zu der Abgabeelementnabe (11) drehfest und axial bewegbar ist; wobei der Adapter (2; 2'; 2") innerhalb der rohrförmigen Kappe (3; 3'; 3") eingerichtet ist; wobei der Adapter eine distal gerichtete Oberfläche (20c; 21a") umfasst;
wobei der Abgabeelementschutz (4) eine proximal gerichtete Oberfläche (40a) angrenzend an die distal gerichtete Oberfläche (20c; 21a") des Adapters (2; 2'; 2") umfasst; wobei der Abgabeelementschutz (4) von einer distalen Position in eine proximale Position relativ zu der rohrförmigen Kappe (3; 3'; 3") axial bewegbar ist;
wobei ein Drehmechanismus zwischen der rohrförmigen Kappe (3; 3'; 3") und dem Adapter (2; 2'; 2") eingerichtet ist; und wobei der Drehmechanismus konfiguriert ist, sodass eine Drehung des Adapters (2; 2'; 2") um die Längsachse (L) herum relativ zu der rohrförmigen Kappe (3; 3'; 3") ausgelöst wird, wenn sich der Abgabeelementschutz (4) von der distalen Position in die proximale Position bewegt, sodass die Abgabeelementnabe (11) von der nicht angebrachten Position in die angebrachte Position durch die Drehung des Adapters (2; 2'; 2") bewegt wird.

2. Unteranordnung nach Anspruch 1, wobei der Drehmechanismus ein Schraubengewinde (20a; 30a') und einen Vorsprung (31b), der innerhalb des Schraubengewindes (20a; 30a') positioniert ist, umfasst; wobei sich eines von dem Schraubengewinde (20a; 30a') und dem Vorsprung (31b) von dem Körper (20; 20') des Adapters (2; 2') erstreckt; und wobei sich das andere eine von dem Schraubengewinde (20a; 30a') und dem Vorsprung (31b) von einem Körper (30; 30') der rohrförmigen Kappe (3; 3') zu dem Körper (20; 20') des Adapters (2; 2') hin erstreckt.

3. Unteranordnung nach Anspruch 2, wobei das Schraubengewinde (20a) an einer Außenoberfläche des Körpers (20) des Adapters (2) eingerichtet ist; wobei die rohrförmige Kappe (3) einen sich distal erstreckenden Arm (31a), der sich von dem Körper (30) der rohrförmigen Kappe in der Richtung der Längsachse (L) erstreckt, umfasst; und wobei sich der Vorsprung (31b) in einer Richtung quer zu der Längsachse (L) von dem sich distal erstreckenden Arm (31a) erstreckt.

4. Unteranordnung nach Anspruch 2, wobei das Schraubengewinde (30a') an einer Innenoberfläche des Körpers (30') der rohrförmigen Kappe (3') eingerichtet ist; und wobei sich der Vorsprung in einer Richtung quer zu der Längsachse (L) von einer Außenoberfläche des Körpers (20') des Adapters (2') erstreckt.

5. Unteranordnung nach Anspruch 1, wobei der Drehmechanismus eine Torsionsfeder (5), die sich um die Längsachse (L) herum windet, umfasst; wobei sich die Torsionsfeder (5) zwischen einem ersten Ende (51) und einem zweiten Ende (52) erstreckt; wobei das erste Ende (51) an der rohrförmigen Kappe (3") fixiert ist; wobei das zweite Ende (52) an dem Körper (20") des Adapters (2') fixiert ist; wobei der Adapter (2") ein Eingriffselement (22), das sich von dem Körper (20") des Adapters (2") erstreckt, umfasst; und wobei die rohrförmige Kappe (3") ein Gegeneingriffselement (31b") umfasst, das mit dem Eingriffselement (22) des Adapters (2") in einer Umfangsrichtung ausgerichtet ist, wenn sich der Abgabeelementschutz (4) in der distalen Position befindet, und relativ zu dem Eingriffselement (22) des Adapters (2") in der Umfangsrichtung versetzt ist, wenn sich der Abgabeelementschutz (4) in der proximalen Position befindet.

6. Unteranordnung nach Anspruch 5, wobei das Eingriffselement (22) eine Rippe, die sich in der Richtung quer zu der Längsachse (L) von einer Außenoberfläche des Körpers (20") des Adapters (2") erstreckt, ist; wobei das Gegeneingriffselement (31b') eine Aussparung in einer Wand des Körpers (30") der rohrförmigen Kappe (3") ist.

7. Unteranordnung nach Anspruch 5 oder 6, wobei der Adapter einen Flansch (21"), der sich von dem Körper (20") des Adapters erstreckt, umfasst; wobei der Flansch (21") die distal gerichtete Oberfläche (21a') definiert; wobei eine Aussparung (21b") in dem Flansch (21") eingerichtet ist und wobei das zweite Ende (52) der Torsionsfeder (5) innerhalb der Aussparung (21b") des Flanschs (21") angebracht ist.

8. Unteranordnung nach einem der vorstehenden Ansprüche, wobei die Abgabeelementanordnung (1) eine rohrförmige Innenkappe (10) umfasst; wobei die Abgabeelementnabe (11) innerhalb der rohrförmigen Innenkappe (10) eingerichtet ist; wobei die Abgabeelementnabe (11) über einen ersten Eingriff zwischen der Abgabeelementnabe (11) und der Innenkappe (10) relativ zu der Innenkappe (10) drehfest und axial bewegbar ist; wobei die Innenkappe (10) innerhalb des Körpers (20; 20'; 20") eingerichtet ist; und wobei die Innenkappe (10) über einen zweiten Eingriff zwischen der Innenkappe (10) und dem Körper (20; 20'; 20") des Adapters (2; 2'; 2") relativ zu dem Körper (20; 20'; 20") des Adapters (2; 2'; 2") drehfest ist.

9. Unteranordnung nach Anspruch 8, wobei der erste Eingriff durch eine sich längs erstreckende Rippe an der Innenkappe und eine sich längs erstreckende Rippe an der Abgabeelementnabe gebildet wird; oder der erste Eingriff durch eine sich längs erstreckende Rippe an der Innenkappe und eine Aussparung/Vertiefung an der Abgabeelementnabe gebildet wird, wobei die Aussparung/Vertiefung in die Richtung quer zu der Längsachse offen ist; oder der erste Eingriff durch eine sich längs erstreckende Rippe an der Abgabeelementnabe und eine Aussparung/Vertiefung an der Innenkappe gebildet wird, wobei die Aussparung/Vertiefung in die Richtung quer zu der Längsachse offen ist; oder der erste Eingriff durch einen Körperabschnitt mit nicht kreisförmigem Querschnitt der Abgabeelementnabe, der mit einem Körperabschnitt mit nicht kreisförmigem Querschnitt der Innenkappe zusammenpasst, gebildet wird.

10. Unteranordnung nach Anspruch 8 oder 9, wobei der zweite Eingriff durch eine sich längs erstreckende Rippe an der Innenkappe und eine sich längs erstreckende Rippe an dem Körper des Adapters gebildet wird; oder der zweite Eingriff durch eine sich längs erstreckende Rippe an der Innenkappe und eine Aussparung/Vertiefung an dem Körper des Adapters gebildet wird, wobei die Aussparung/Vertiefung in die Richtung quer zu der Längsachse offen ist; oder der zweite Eingriff durch eine sich längs erstreckende Rippe an dem Körper des Adapters und eine Aussparung/Vertiefung an der Innenkappe gebildet wird, wobei die Aussparung/Vertiefung in die Richtung quer zu der Längsachse offen ist; oder der zweite Eingriff durch einen Körperabschnitt mit nicht kreisförmigem Querschnitt des Körpers des Adapters, der mit einem Körperabschnitt mit nicht kreisförmigem Querschnitt der Innenkappe zusammenpasst, gebildet wird.

11. Unteranordnung nach einem der Ansprüche 1 bis 7, wobei der Adapter mit der Abgabeelementnabe direkt in Eingriff steht, über einen Eingriff, der gebildet wird durch: eine sich längs erstreckende Rippe an der Abgabeelementnabe und eine sich längs erstreckende Rippe an dem Körper des Adapters; oder der Eingriff durch eine sich längs erstreckende Rippe an der Abgabeelementnabe und eine Aussparung/Vertiefung an dem Körper des Adapters gebildet wird, wobei die Aussparung/Vertiefung in die Richtung quer zu der Längsachse offen ist; oder der Eingriff durch eine sich längs erstreckende Rippe an dem Körper des Adapters und eine Aussparung/Vertiefung an der Abgabeelementnabe gebildet wird, wobei die Aussparung/Vertiefung in die Richtung quer zu der Längsachse offen ist; oder der Eingriff durch einen Körperabschnitt mit nicht kreisförmigem Querschnitt des Körpers des Adapters, der mit einem Körperabschnitt mit nicht kreisförmigem Querschnitt der Abgabeelementnabe zusammenpasst, gebildet wird.

12. Unteranordnung nach einem der vorstehenden Ansprüche, wobei eines von dem Befestigungsmittel und dem Gegenbefestigungsmittel ein Schraubengewinde ist und das andere eine von dem Befestigungsmittel und dem Gegenbefestigungsmittel ein Schraubengewindefolger ist; und wobei die Abgabeelementnabe in der distalen Richtung der rohrförmigen Kappe relativ zu dem Gehäuse der Medikamentenabgabevorrichtung axial bewegbar ist.

13. Unteranordnung nach einem der vorstehenden Ansprüche, wobei der Abgabeelementschutz (4) einen rohrförmigen Körper (40) umfasst, der konfiguriert ist, um das Abgabeelement (12) zu umgeben; und wobei das proximale Ende des rohrförmigen Körpers (40) die proximal gerichtete Oberfläche (40a) definiert.

14. Vordere Unteranordnung für eine Medikamentenabgabevorrichtung, die vordere Unteranordnung umfassend die Unteranordnung nach einem der vorstehenden Ansprüche; wobei die vordere Unteranordnung ferner umfasst:
einen Körper (60), der sich entlang der Längsachse (L) von einem proximalen Ende zu einem distalen Ende erstreckt;
den Abgabeelementschutz (4), der an dem Körper (60) koaxial angebracht ist und entlang der Längsachse (L) relativ zu dem Körper (60) axial bewegbar ist; und ein Verriegelungselement (62), das an dem Körper (20) angebracht ist;
wobei das Verriegelungselement (62) zwischen einer verriegelten Position und einer entriegelten Position relativ zu dem Abgabeelementschutz (4) bewegbar ist; und
wobei das Verriegelungselement (62) in der verriegelten Position eine distal gerichtete Oberfläche angrenzend an eine zweite proximal gerichtete Oberfläche (43a) des Abgabegliedschutzes (4) umfasst; und wobei die distal gerichtete Oberfläche des Verriegelungselements (62) in der entriegelten Position in einer Umfangsrichtung relativ zu der Längsachse (L) von der proximal gerichteten Oberfläche des Abgabeelementschutzes (4) beabstandet ist.

15. Vordere Unteranordnung nach Anspruch 15, wobei das Verriegelungselement (62) zwischen der verriegelten Position und der entriegelten Position relativ zu dem Abgabeelementschutz (4) um die Längsachse (L) herum drehbar ist.

## Revendications

1. Sous-ensemble d'un dispositif d'administration de médicament , le sous-ensemble comprenant :
un capuchon tubulaire (3 ; 3' ; 3"), un adaptateur (2 ; 2' ; 2"), un ensemble d'élément d'administration (1) et un protecteur d'élément d'administration (4) ;
dans lequel le capuchon tubulaire (3 ; 3' ; 3") s'étend le long d'un axe longitudinal (L) entre une extrémité proximale et une extrémité distale ;
dans lequel l'ensemble d'élément d'administration (1) comprend une embase d'élément d'administration (11) et un élément d'administration (12) fixé à l'embase d'élément d'administration (11) ; dans lequel l'embase d'élément d'administration (11) comprend une attache (112) conçue pour être fixée à une contre-attache (132) d'un boîtier du dispositif d'administration de médicament par une rotation relative entre l'attache (112) et la contre-attache (132) ; dans lequel l'embase d'élément d'administration (11) peut tourner par rapport au boîtier du dispositif d'administration de médicament autour de l'axe longitudinal (L) entre une position non fixée où l'attache (112) n'est pas fixée à la contre-attache (132) et une position fixée où l'attache (112) est fixée à la contre-attache (132) ;
dans lequel l'adaptateur (2 ; 2' ; 2") comprend un corps (20 ; 20' ; 20") relié de manière fonctionnelle à l'embase d'élément d'administration (11), de sorte que l'adaptateur (2 ; 2' ; 2") est immobile en rotation et mobile axialement par rapport à l'embase d'élément d'administration (11) ; dans lequel l'adaptateur (2 ; 2' ; 2") est agencé à l'intérieur du capuchon tubulaire (3 ; 3' ; 3") ; dans lequel l'adaptateur comprend une surface orientée distalement (20c ; 21a") ;
dans lequel le protecteur d'élément d'administration (4) comprend une surface orientée proximalement (40a) adjacente à la surface orientée distalement (20c ; 21a") de l'adaptateur (2 ; 2' ; 2") ; dans lequel le protecteur d'élément d'administration (4) est mobile axialement par rapport au capuchon tubulaire (3 ; 3' ; 3") depuis une position distale vers une position proximale ;
dans lequel un mécanisme de rotation est agencé entre le capuchon tubulaire (3 ; 3' ; 3") et l'adaptateur (2 ; 2' ; 2") ; et dans lequel le mécanisme de rotation est conçu de sorte qu'une rotation de l'adaptateur (2 ; 2' ; 2") autour de l'axe longitudinal (L) par rapport au capuchon tubulaire (3 ; 3' ; 3") est déclenchée lorsque le protecteur d'élément d'administration (4) se déplace de la position distale à la position proximale, de sorte que l'embase d'élément d'administration (11) est déplacée de la position non fixée à la position fixée par la rotation de l'adaptateur (2 ; 2' ; 2").

2. Sous-ensemble selon la revendication 1, dans lequel le mécanisme de rotation comprend un filetage (20a ; 30a') et une protubérance (31b) positionnée à l'intérieur du filetage (20a ; 30a') ; dans lequel l'un parmi le filetage (20a ; 30a') et la protubérance (31b) s'étend depuis le corps (20 ; 20') de l'adaptateur (2 ; 2') ; et dans lequel l'autre parmi le filetage (20a ; 30a') et la protubérance (31b) s'étend depuis un corps (30 ; 30') du capuchon tubulaire (3 ; 3') vers le corps (20 ; 20') de l'adaptateur (2 ; 2').

3. Sous-ensemble selon la revendication 2, dans lequel le filetage (20a) est agencé sur une surface externe du corps (20) de l'adaptateur (2) ; dans lequel le capuchon tubulaire (3) comprend un bras s'étendant distalement (31a) s'étendant depuis le corps (30) du capuchon tubulaire dans la direction de l'axe longitudinal (L) ; et dans lequel la protubérance (31b) s'étend dans une direction transversale à l'axe longitudinal (L) depuis le bras s'étendant distalement (31a).

4. Sous-ensemble selon la revendication 2, dans lequel le filetage (30a') est agencé sur une surface interne du corps (30') du capuchon tubulaire (3') ; et dans lequel la protubérance s'étend dans une direction transversale à l'axe longitudinal (L) depuis une surface externe du corps (20') de l'adaptateur (2').

5. Sous-ensemble selon la revendication 1, dans lequel le mécanisme de rotation comprend un ressort de torsion (5) s'enroulant autour de l'axe longitudinal (L) ; dans lequel le ressort de torsion (5) s'étend entre une première extrémité (51) et une seconde extrémité (52) ; dans lequel la première extrémité (51) est fixée au capuchon tubulaire (3") ; dans lequel la seconde extrémité (52) est fixée au corps (20") de l'adaptateur (2') ; dans lequel l'adaptateur (2") comprend un élément de mise en prise (22) s'étendant depuis le corps (20") de l'adaptateur (2") ; et dans lequel le capuchon tubulaire (3") comprend un contre-élément de mise en prise (31b") aligné avec l'élément de mise en prise (22) de l'adaptateur (2") dans une direction circonférentielle lorsque le protecteur d'élément d'administration (4) est dans la position distale, et décalé par rapport à l'élément de mise en prise (22) de l'adaptateur (2") dans la direction circonférentielle lorsque le protecteur d'élément d'administration (4) est dans la position proximale.

6. Sous-ensemble selon la revendication 5, dans lequel l'élément de mise en prise (22) est une nervure s'étendant dans la direction transversale à l'axe longitudinal (L) depuis une surface externe du corps (20") de l'adaptateur (2") ; dans lequel le contre-élément de mise en prise (31b"') est une découpe dans une paroi du corps (30") du capuchon tubulaire (3").

7. Sous-ensemble selon la revendication 5 ou 6, dans lequel l'adaptateur comprend une bride (21") s'étendant depuis le corps (20") de l'adaptateur ; dans lequel la bride (21") définit la surface orientée distalement (21a') ; dans lequel une découpe (21b") est agencée dans la bride (21") et dans lequel la seconde extrémité (52) du ressort de torsion (5) est fixée à l'intérieur de la découpe (21b") de la bride (21").

8. Sous-ensemble selon l'une quelconque des revendications précédentes, dans lequel l'ensemble d'élément d'administration (1) comprend un capuchon interne tubulaire (10) ; dans lequel l'embase d'élément d'administration (11) est agencée à l'intérieur du capuchon interne tubulaire (10) ; dans lequel l'embase d'élément d'administration (11) est immobile en rotation et mobile axialement par rapport au capuchon interne (10) par l'intermédiaire d'une première mise en prise entre l'embase d'élément d'administration (11) et le capuchon interne (10) ; dans lequel le capuchon interne (10) est agencé à l'intérieur du corps (20 ; 20' ; 20") ; et dans lequel le capuchon interne (10) est immobile en rotation par rapport au corps (20 ; 20' ; 20") de l'adaptateur (2 ; 2' ; 2") par l'intermédiaire d'une seconde mise en prise entre le capuchon interne (10) et le corps (20 ; 20' ; 20") de l'adaptateur (2 ; 2' ; 2").

9. Sous-ensemble selon la revendication 8, dans lequel la première mise en prise est formée par une nervure s'étendant longitudinalement sur le capuchon interne et une nervure s'étendant longitudinalement sur l'embase d'élément d'administration ; ou la première mise en prise est formée par une nervure s'étendant longitudinalement sur le capuchon interne et une découpe/un évidement sur l'embase d'élément d'administration, dans lequel la découpe/l'évidement est ouvert(e) vers la direction transversale à l'axe longitudinal ; ou la première mise en prise est formée par une nervure s'étendant longitudinalement sur l'embase d'élément d'administration et une découpe/un évidement sur le capuchon interne, dans lequel la découpe/l'évidement est ouvert(e) vers la direction transversale à l'axe longitudinal ; ou la première mise en prise est formée par une section de corps à section transversale non circulaire de l'embase d'élément d'administration correspondant à une section de corps à section transversale non circulaire du capuchon interne.

10. Sous-ensemble selon la revendication 8 ou 9, dans lequel la seconde mise en prise est formée par une nervure s'étendant longitudinalement sur le capuchon interne et une nervure s'étendant longitudinalement sur le corps de l'adaptateur ; ou la seconde mise en prise est formée par une nervure s'étendant longitudinalement sur le capuchon interne et une découpe/un évidement sur le corps de l'adaptateur, dans lequel la découpe/l'évidement est ouvert(e) vers la direction transversale à l'axe longitudinal ; ou la seconde mise en prise est formée par une nervure s'étendant longitudinalement sur le corps de l'adaptateur et une découpe/un évidement sur le capuchon interne, dans lequel la découpe/l'évidement est ouvert(e) vers la direction transversale à l'axe longitudinal ; ou la seconde mise en prise est formée par une section de corps à section transversale non circulaire du corps de l'adaptateur correspondant à une section de corps à section transversale non circulaire du capuchon interne.

11. Sous-ensemble selon l'une quelconque des revendications 1 à 7, dans lequel l'adaptateur est directement mis en prise avec l'embase d'élément d'administration par l'intermédiaire d'une mise en prise formée par : une nervure s'étendant longitudinalement sur l'embase d'élément d'administration et une nervure s'étendant longitudinalement sur le corps de l'adaptateur ; ou la mise en prise est formée par une nervure s'étendant longitudinalement sur l'embase d'élément d'administration et une découpe/un évidement sur le corps de l'adaptateur, dans lequel la découpe/l'évidement est ouvert(e) vers la direction transversale à l'axe longitudinal ; ou la mise en prise est formée par une nervure s'étendant longitudinalement sur le corps de l'adaptateur et une découpe/un évidement sur l'embase d'élément d'administration, dans lequel la découpe/l'évidement est ouvert(e) vers la direction transversale à l'axe longitudinal ; ou la mise en prise est formé(e) par une section de corps à section transversale non circulaire du corps de l'adaptateur correspondant à une section de corps à section transversale non circulaire de l'embase d'élément d'administration.

12. Sous-ensemble selon l'une quelconque des revendications précédentes, dans lequel l'une parmi l'attache et la contre-attache est un filetage, et l'autre parmi l'attache et la contre-attache est un suiveur de filetage ; et dans lequel l'embase d'élément d'administration est mobile axialement par rapport au boîtier du dispositif d'administration de médicament dans la direction distale du capuchon tubulaire.

13. Sous-ensemble selon l'une quelconque des revendications précédentes, dans lequel le protecteur d'élément d'administration (4) comprend un corps tubulaire (40) conçu pour entourer l'élément d'administration (12) ; et dans lequel l'extrémité proximale du corps tubulaire (40) définit la surface orientée proximalement (40a).

14. Sous-ensemble avant pour un dispositif d'administration de médicament, le sous-ensemble avant comprenant le sous-ensemble selon l'une quelconque des revendications précédentes ; dans lequel le sous-ensemble avant comprend en outre :
- un corps (60) s'étendant le long d'un axe longitudinal (L) d'une extrémité proximale à une extrémité distale ;
le protecteur d'élément d'administration (4) fixé coaxialement au corps (60) et étant mobile axialement par rapport au corps (60) le long de l'axe longitudinal (L) ; et un élément de verrouillage (62) fixé au corps (20) ;
dans lequel l'élément de verrouillage (62) est mobile par rapport au protecteur d'élément d'administration (4) entre une position verrouillée et une position déverrouillée ; et
dans lequel l'élément de verrouillage (62) comprend une surface orientée distalement adjacente à une seconde surface orientée proximalement (43a) du protecteur d'élément d'administration (4) dans la position verrouillée ; et dans lequel la surface orientée distalement de l'élément de verrouillage (62) est espacée dans une direction circonférentielle par rapport à l'axe longitudinal (L) de la surface orientée proximalement du protecteur d'élément d'administration (4) dans la position déverrouillée.

15. Sous-ensemble avant selon la revendication 15, dans lequel l'élément de verrouillage (62) peut tourner par rapport au protecteur d'élément d'administration (4) autour de l'axe longitudinal (L) entre la position verrouillée et la position déverrouillée.
